# EUROPÄISCHE PATENTSCHRIFT

(11) **EP 3 662 868 B1**
(45) Veröffentlichungstag und Bekanntmachung des Hinweises auf die Patenterteilung: **24.03.2021**
(21) Anmeldenummer: 18210881.1
(22) Anmeldetag: 06.12.2018
(51) Int. Cl.: A61F 2/30, A61F 2/44

(54) **ZWISCHENWIRBELIMPLANTAT UND VERFAHREN ZUR HERSTELLUNG EINES ZWISCHENWIRBELIMPLANTATS**
INTERVERTEBRAL JOINT AND METHOD FOR PRODUCING SAME
IMPLANT INTERVERTÉBRAL ET PROCÉDÉ DE FABRICATION D'UN IMPLANT INTERVERTÉBRAL

(43) Veröffentlichungstag der Anmeldung: 10.06.2020
(73) Patentinhaber: Kraus, Kilian, 97440 Werneck (DE)
(72) Erfinder: Kraus, Kilian, 97440 Werneck (DE)
(74) Vertreter: Schlögl, Markus

(56) Entgegenhaltungen:
- WO-A1-2009/092102
- US-A1- 2010 049 325
- US-A1- 2011 029 085
- US-A1- 2011 224 796
- US-A1- 2013 158 663

## Beschreibung

Die Erfindung betrifft ein Zwischenwirbelimplantat, ein sogenannter Cage, insbesondere zur Verwendung im Rahmen einer ALIF- (anterior lumbar interbody fusion), PLIF- (posterior lumbar interbody fusion), TLIF- (transforaminal lumbar interbody fusion) oder XLIF- (extreme lateral interbody fusion) Operation, bei der Wirbelkörper miteinander verschmolzen werden.

Aus dem Stand der Technik sind sowohl einstückige als auch mehrteilige Implantate für den Wirbelsäulenbereich bekannt, die beispielsweise im Rahmen einer Versteifungsoperation der Wirbelsäule, insbesondere einer lumbalen interkorporellen Fusion, also einer Wirbelkörperfusion im Lendenwirbelbereich, Verwendung finden. Einstückige bzw. massive Zwischenwirbelimplantate haben im Allgemeinen den Vorteil erhöhter mechanischer Stabilität. Zudem können derartige Implantate porös oder mit Durchgängen versehen sein, um ein Durchwachsen mit Knochenmaterial und/oder ein Verfüllen mit Knochenersatzmaterial, damit das Zwischenwirbelimplantat nicht im Knochen einsinkt, zu ermöglichen. Aus WO 2009/068021 A1 ist beispielsweise ein Zwischenwirbelimplantat mit einer inneren Kanalstruktur bekannt, welches ein Einwachsen von Knochenmaterial begünstigt.

Aufgrund von anatomischen Unterschieden, insbesondere hinsichtlich der Dicke und Ausrichtung der Bandscheiben in der Wirbelsäule, müssen bei einer Operation zur intersomatischen oder interkorporellen Wirbelkörperfusion eine große Anzahl, meist in etwa 100 Stück, von einstückigen bzw. massiven Zwischenwirbelimplantaten in unterschiedlichen Ausgestaltungen verfügbar sein. Derartige Zwischenwirbelimplantate unterscheiden sich hinsichtlich ihrer Gestalt, insbesondere hinsichtlich ihrer Höhen- oder Längsausdehnung und/oder hinsichtlich der Ausrichtung von zur Anlage an die Wirbelkörper vorgesehenen Kontaktoberflächen oftmals nur geringfügig, so dass sich die Auswahl des zum Implantieren optimal angepassten Implantats für den behandelnden Arzt als schwierig gestalten kann.

Die bereits genannten Operationstechniken (ALIF, PLIF, TLIF, XLIF) unterscheiden sich im Wesentlichen in der Art des Zugangswegs zur Wirbelsäule. So erfolgt beispielsweise das Einsetzen eines Zwischenwirbelimplantats bei einer PLIF-Operation dorsal, also vom Rücken her. Aufgrund der Ausrichtung insbesondere von Wirbeln im unteren Lendenwirbelbereich bedingt dies die Verwendung von in etwa keilförmig ausgebildeten Zwischenwirbelimplantaten, die allerdings aufgrund der anatomischen Begebenheiten mit der breiteren Seite zuerst eingesetzt werden müssen. Bei der Verwendung von einstückigen Zwischenwirbelimplantaten kann dies mit einer erhöhten Gefahr insbesondere einer neuronalen Verletzung einhergehen.

Zur Umgehung dieser Probleme wurden im Stand der Technik bereits mehrteilige, expandierbare und insbesondere hinsichtlich der Bauhöhe verstellbare Implantate vorgeschlagen. So beschreibt WO 2009/092102 A1 beispielsweise ein expandierbares Zwischenwirbelimplantat, welches insbesondere hinsichtlich seiner Vertikalausdehnung variabel ist. Das Zwischenwirbelimplantat ist dazu ausgebildet, im kollabierten Zustand eingesetzt zu werden, um anschließend auf die Höhe des entfernten Bandscheibenmaterials expandiert zu werden. WO 2008/070863 A2 beschreibt ein höhenverstellbares Zwischenwirbelimplantat, welches mit Hilfe einer keilförmigen Führung an die Abmessung des auszufüllenden Zwischenwirbelbereichs angepasst werden kann.

US 2013/0158663 A1 beschreibt ein Zwischenwirbelimplantat mit ersten und zweiten, zur Anlage Wirbelkörpern vorgesehene, Abstützelemente. Die Abstützelemente können zueinander mit Hilfe einer dazwischen angeordneten, keilähnlich ausgebildeten Komponente verstellt werden, die zumindest zwei, zueinander in Axialrichtung beabstandete Rampen aufweist.

Problematisch an derartigen mehrteiligen Ausführungen ist, dass sie zum einen -zumindest solange das Implantat noch nicht von einer tragfähigen Knochenstruktur durchwachsen ist- dazu in der Lage sein müssen, beträchtlichen Kräften Stand zu halten. Zum anderen ist ein vollständiges Durchwachsen bei gängigen mehrteiligen Zwischenwirbelimplantaten oftmals durch den zur Höhenverstellung vorgesehenen Spreizmechanismus erschwert, der einen großen Teil des mit Knochenmaterial zu verfüllenden Innenvolumens beanspruchen kann. Selbst im eingewachsenen Zustand wird daher oftmals keine zufriedenstellende Tragfähigkeit erreicht.

Die Erfindung stellt sich zur Aufgabe, ein hinsichtlich seiner geometrischen Gestalt variabel anpassbares Zwischenwirbelimplantat anzugeben, welches insbesondere eine hohe mechanische Stabilität aufweist.

Diese Aufgabe wird gelöst durch ein Zwischenwirbelimplantat mit den Merkmalen des Patentanspruchs 1.

Vorteilhafte Ausgestaltungen sind Gegenstand der Unteransprüche.

Ein Zwischenwirbelimplantat (auch: Cage) weist zwei gegenüberliegend angeordnete, zur zumindest abschnittsweisen Anlage an Wirbelkörpern ausgebildete Kontaktoberflächen auf, die voneinander entlang einer Hochachse beabstandet sind. Die Kontaktoberflächen sind jeweils auf relativ zueinander verstellbaren Abstützelementen angeordnet, die entlang einer senkrecht zur Hochachse verlaufenden Längsachse orientierten Kreisbogenkontur derart zueinander verstellbar geführt sind, dass durch Verstellen der beiden Abstützelemente relativ zueinander entlang der Kreisbogenkontur ein Abstand der beiden Kontaktoberflächen bezüglich der Hochachse und eine Winkelstellung der beiden Kontaktoberflächen zueinander und bezüglich der Hochachse vorgegeben und/oder verändert werden kann.

Unter der Wendung, dass die beiden Abstützelemente entlang einer Kreisbogenkontur zueinander verstellbar sind, soll insbesondere verstanden werden, dass die beiden Abstützelemente entlang der Kreisbogenkontur verschiebbar geführt sind und zumindest an vorgegebenen Positionen zueinander arretiert werden können. Bevorzugt sind die beiden Abstützelemente zueinander an beliebiger Position arretierbar. Die Arretierung kann lösbar (auch: reversibel) oder unlösbar (auch: irreversibel) sein.

Unter der Wendung, dass die Kreisbogenkontur entlang einer senkrecht zur Hochachse verlaufenden Längsachse orientiert ist, soll insbesondere verstanden werden, dass die Krümmung der Kreisbogenkontur in einer Ebene verläuft, die parallel zur der von der Hoch- und der Längsachse aufgespannten Ebene verläuft. In Ausgestaltungen ist die Hochachse beispielsweise identisch mit der Mittelhochachse des Zwischenwirbelimplantats und die Längsachse insbesondere mit der Mittellängsachse des Zwischenwirbelimplantats.

Da die beiden Abstützelemente entlang der Kreisbogenkontur zueinander verstellbar sind, die entlang der Längsachse des Zwischenwirbelimplantats orientiert ist, bewirkt ein Verstellen der beiden Abstützelemente zueinander neben einer Variation der Längenausdehnung im Allgemeinen sowohl eine Veränderung der Höhenausdehnung, also eine Veränderung des Abstands der beiden gegenüberliegenden Kontaktoberflächen zueinander, als auch eine Veränderung des Winkels, in dem die Kontaktoberflächen zueinander angeordnet sind. Auf diese Weise ist ermöglicht, dass das gleiche Zwischenwirbelimplantat für unterschiedliche Implantationsorte verwendet werden kann. Insbesondere kann das erfindungsgemäße Zwischenwirbelimplantat im Rahmen einer intersomatischen oder interkorporellen Wirbelkörperfusion an den Abstand und die Ausrichtung der beiden zu überbrückenden bzw. zu fusionierenden Wirbelkörper flexibel angepasst werden. Im Ergebnis kann daher die Anzahl der für eine derartige Operation zur Verfügung zu stellenden Zwischenwirbelimplantate erheblich reduziert werden, was letztendlich auch dem behandelnden Arzt die Wahl des an den Implantationsort optimal angepassten Zwischenwirbelimplantats erleichtert.

Die Kreisbogenkontur kann in verschiedenen Ausgestaltungen des Zwischenwirbelimplantats unterschiedlich verlaufen. Insbesondere sind Variationen hinsichtlich des Krümmungsradius und/oder der Neigung der Kreisbogenkontur bezüglich der Längsachse und/oder der Hochachse des Zwischenwirbelimplantats möglich und vorgesehen. Die Krümmung und Neigung der Kreisbogenkontur definiert insbesondere, inwieweit eine Längenänderung des Zwischenwirbelimplantats mit einer Änderung der Höhenausdehnung bzw. der Ausrichtung (auch: Winkelstellung) der Kontaktoberflächen zueinander einhergeht. Es ist vorgesehen, insbesondere diesbezüglich gegenständlich unterschiedlich ausgestaltete Varianten des erfindungsgemäßen Zwischenwirbelimplantats dem behandelnden Arzt bei der Operation zur Verfügung zu stellen.

Durch Verstellen der beiden Abstützelemente wird eine Variation der Länge des Zwischenwirbelimplantats in Richtung der Längsachse und/oder eine Variation der Höhe bzw. Dicke des Zwischenwirbelimplantats in Richtung der Hochachse und/oder eine Variation der Ausrichtung der beiden Kontaktoberflächen zueinander bewirkt. Bei typischen Ausgestaltungen und Auslegungen des Zwischenwirbelimplantats und insbesondere des Verlaufs der Kreisbogenkontur liegt die Länge des Zwischenwirbelimplantats in etwa zwischen 15mm und 45mm, bevorzugt in etwa zwischen 20mm und 40mm, d. h. die Länge kann maximal in etwa um 30mm, bevorzugt in etwa um 20mm variieren. Entsprechend liegt die Dicke (auch: Höhe) des Zwischenwirbelimplantats bei typischen Auslegungen zwischen 4mm und 22mm, bevorzugt zwischen 6mm und 20mm, d. h. die Variation in Richtung der Hochachse beträgt maximal in etwa 18mm, bevorzugt in etwa 14mm. Die Kontaktoberflächen erstrecken sich parallel zur Längsachse oder verlaufen bezüglich der Längsachse von bis zu ±15°. Entsprechend liegt die maximale Variation der Ausrichtung der beiden Kontaktoberflächen zueinander in typischen Ausgestaltungen bei etwa 30°.

Die Kreisbogenkontur weist insbesondere einen konstanten Krümmungsradius auf. Durch eine derartige Ausbildung wird erreicht, dass die beiden Abstützelemente stets entlang der Kreisbogenkontur eng aneinander anliegen. Dies ist insbesondere vorteilhaft, um große, in Richtung der Hochachse wirkende Kräfte zu kompensieren.

Das Zwischenwirbelimplantat ist insbesondere dazu vorgesehen, derart in einen Bereich zwischen zwei Wirbelkörpern eingesetzt zu werden, dass die Hochachse in Richtung der Wirbelsäulenlängsachse orientiert ist. Durch ein Verstellen der beiden Abstützelemente entlang der Kreisbogenkontur kann die Ausdehnung des Zwischenwirbelimplantats insbesondere in Richtung der Wirbelsäulenlängsachse auf die Höhe des zu ersetzenden Bandscheibenmaterials angepasst werden. Zudem bewirkt das Verstellen der beiden Abstützelemente zueinander eine Veränderung der gegenüberliegend angeordneten Kontaktoberflächen. Die Neigung der beiden Kontaktoberflächen wird vorzugsweise derart an die Ausrichtung der beiden angrenzenden Wirbelkörper angepasst, dass diese über einen möglichst großen Bereich an den jeweiligen Kontaktoberflächen anliegen.

Da das Zwischenwirbelimplantat hinsichtlich der Bauhöhe, also hinsichtlich seiner Ausdehnung in Richtung der Hochachse, variabel ausgestaltet ist, ist insbesondere ermöglicht, die Bauhöhe vor dem Einsetzen zu minimieren, damit zum Implantieren lediglich minimale Zugangswege erforderlich sind. Die Anpassung der räumlichen Gestalt des Zwischenwirbelimplantats, insbesondere des Abstandes und/oder der Ausrichtung der Kontaktoberflächen zueinander durch Verstellen der beiden Abstützelemente, an die anatomischen Begebenheiten des Implantationsorts kann insbesondere nach dem Einsetzen in den menschlichen Körper erfolgen.

Das Zwischenwirbelimplantat ist beispielsweise als knochenverbindendes oder knochenüberbrückendes Implantat ausgestaltet. Hierunter wird insbesondere verstanden, dass zumindest eine Kontaktoberfläche des Zwischenwirbelimplantats zumindest abschnittsweise direkt mit einem Knochen, beispielsweise mit einem Wirbelkörper in Berührung kommt. Die Kontaktoberflächen sind somit dazu vorgesehen, in direktem Kontakt mit Knochenmaterial, insbesondere mit Wirbelkörpern zu stehen und weisen hierzu beispielsweise eine geeignete Rauigkeit und/oder eine Strukturierung, wie etwa eine geriffelte oder mit Zähnen versehene Oberflächenstrukturierung aufweisen. Alternativ oder zusätzlich sind die Kontaktoberflächen beispielsweise porös, insbesondere offenporig porös, um ein Einwachsen von Knochenmaterial zu ermöglichen. In anderen Ausgestaltungen sind die Kontaktoberflächen im Wesentlichen glatt ausgebildet.

In Ausgestaltungen ist die Relativbewegung der beiden Abstützelemente zueinander auf den tangential zur Kreisbogenkontur gerichteten Bewegungsfreiheitsgrad beschränkt. Dies kann beispielsweise durch entsprechende Führungen, die gegebenenfalls Hinterschneidungen aufweisen, bewirkt werden. In Ausgestaltungen weist ein Abstützelement beispielsweise zumindest eine Nut auf, die sich über zumindest einen Abschnitt der Kreisbogenkontur erstreckt. Das andere Abstützelement weist entsprechend zumindest einen Vorsprung auf, der innerhalb der zumindest einen Nut verschiebbar geführt ist.

Zwischenwirbelimplantate, die dazu ausgestaltet sind, mit Knochenmaterial (auch: natürliches Knochenmaterial) zu verwachsen oder mit Knochenersatzmaterial (auch: künstliches Knochenmaterial) ausgefüllt zu werden, weisen vorzugsweise einen Hohlraum bzw. mehrere Hohlräume insbesondere im Bereich zwischen den gegenüberliegend angeordneten Kontaktoberflächen auf.

In bevorzugten Ausgestaltungen umfasst zumindest eines der Abstützelemente eine innere Struktur mit einer Vielzahl von zur Kontaktoberfläche offenen Kanälen. Die innere Struktur (auch: Kanalstruktur) erstreckt sich von der am Knochen anliegenden Kontaktoberfläche in das Innere des Zwischenwirbelimplantats. Die Kanäle weisen jeweils eine Querschnittsfläche von 8.000 µm² bis 7000000 µm², bevorzugt eine Querschnittsfläche von 50.000 µm² bis 3.100.000 µm², besonders bevorzugt eine Querschnittsfläche von 125.000 µm² bis 570.000 µm² auf. Eine derartig dimensionierte Kanalstruktur ist an die Kapillarwirkung von Blut angepasst und fördert somit das Eindringen von Blut in ausreichender Tiefe. Dadurch wird in vorteilhafter Weise das Verwachsen des Zwischenwirbelimplantats mit anliegenden Knochen, insbesondere Wirbelkörpern unterstützt.

In Ausgestaltungen sind beide gegenüberliegend angeordnete Kontaktoberflächen mit inneren Strukturen versehen, die jeweils eine Vielzahl von Kanälen umfassen. Die gegenüberliegend angeordneten inneren Strukturen bilden in zumindest einem Teil des Zwischenwirbelimplantats zumindest eine in Richtung der Hochachse insbesondere für Fluide durchlässige Hohlraumstruktur. Derartige Ausführung begünstigen somit das zumindest teilweise, bevorzugt vollständige Durchwachsen des Zwischenwirbelimplantats mit natürlichem Knochenmaterial bzw. das zumindest teilweise, bevorzugt vollständige Verfüllen des Zwischenwirbelimplantats mit künstlichem Knochenmaterial.

In exemplarischen Ausgestaltungen erstrecken sich die von den Kanälen gebildeten inneren Strukturen über die gesamte Ausdehnung des jeweiligen Abstützelements in Richtung der Hochachse. Die Kanäle erstrecken sich beispielsweise im Wesentlichen zumindest abschnittsweise oder vollständig parallel zur Hochachse. Die Wendung, dass die Kanäle im Wesentlichen parallel zur Hochachse verlaufen, ist insbesondere so zu verstehen, dass die mit dem Verstellen der beiden Abstützelemente zueinander einhergehende geringfügige Veränderung der Ausrichtung der Kanäle mitumfasst ist. In Abhängigkeit der relativen Lage der beiden Abstützelemente zueinander können die in den jeweiligen Abstützelementen eingebrachten Kanäle zueinander versetzt angeordnet sein. Vorzugsweise überlappen sich die Kanäle bzw. Kanalstrukturen derart, dass die gebildete Hohlraumstruktur in Richtung der Hochachse durchlässig ist. Hierbei hat es sich als vorteilhaft erwiesen, wenn die Dicke von nebeneinanderliegende Kanäle trennenden Kanalwänden deutlich kleiner ist als der Durchmesser, insbesondere bei Kanälen mit nicht rundem Querschnitt der mittlere Durchmesser der Kanäle. Beispielsweise beträgt der mittlere Durchmesser der Kanäle das 2-fache bis 4-fache der Dicke (auch: Stärke, Kanalwandstärke) der Kanalwände.

In Ausgestaltungen ist die innere Struktur wabenförmig, gitter- oder netzartig ausgebildet. Derartige Ausgestaltungen ermöglichen das Eindringen von natürlichem Knochenmaterial und/oder das Einbringen von Knochenersatzmaterial und sind mechanisch hoch beanspruchbar.

In Ausgestaltungen weisen die Kanäle einen runden oder ovalen Querschnitt oder einen Querschnitt in der Form eines Polygons, insbesondere eines regelmäßigen Polygons, beispielsweise einen dreieckigen, rechteckigen, quadratischen oder hexagonalen Querschnitt auf. Die innere Struktur ist insbesondere durch mehrere solcher Kanäle wabenförmig, gitter- oder netzartig ausgebildet. Insbesondere Kanalstrukturen, die von Kanälen mit polygonalem Querschnitt gebildet sind, weisen eine hohe mechanische Stabilität auf.

In Ausgestaltungen weist zumindest eines der Abstützelemente zumindest eine Kavität auf, die eine Öffnung auf der Kontaktoberfläche des zumindest einen Abstützelements bildet, deren Oberfläche sich zumindest über 1/10 und höchstens 3/4 der Kontaktoberfläche des zumindest einen Abstützelements erstreckt. Die Kavität dient beispielweise dem Eindringen von natürlichem Knochenmaterial oder dem Verfüllen des Zwischenwirbelimplantats mit künstlichem Knochenmaterial bzw. Knochenersatzmaterial. Die Öffnung der Kavität und/oder deren Querschnittsfläche kann hinsichtlich ihrer Form beliebig ausgestaltet sein und ist beispielsweise rund oder oval.

In Ausgestaltungen sind beide gegenüberliegend angeordnete Kontaktoberflächen mit jeweils zumindest einer Kavität versehen, die derartig gegenüberliegend angeordneten sind, dass die Kavitäten in zumindest einem Teil des Zwischenwirbelimplantats zumindest eine in Richtung der Hochachse durchgängigen Hohlraum bilden. Die Größe des in Richtung der Hochachse durchgängigen Hohlraums kann insbesondere von der relativen Anordnung der beiden Abstützelemente zueinander abhängen.

In weiteren Ausgestaltungen weisen beide Abstützelemente jeweils sowohl eine innere Struktur mit mehreren Kanälen als auch zumindest eine Kavität auf, die einen in Richtung der Hochachse durchgängigen Hohlraum bilden. In anderen Ausführungsbeispielen weist ein Abstützelement zumindest eine, relativ große Kavität auf und das andere Abstützelement eine innere Struktur mit mehreren, relativ kleinen Kanälen. Auch auf diese Weise kann eine Hohlraumstruktur in zumindest einem Teil des Zwischenwirbelimplantats geschaffen werden, die in Richtung der Hochachse durchlässig ist.

Die Kontaktoberflächen können eben ausgeführt sein oder eine Krümmung aufweisen. In Ausgestaltungen ist zumindest eine der Kontaktoberflächen konvex gewölbt. Bevorzugt sind die beiden gegenüberliegenden Kontaktoberflächen nach außen konvex gewölbt.

In Ausgestaltungen sind die beiden die Kontaktoberflächen aufweisenden Abstützelemente zueinander mittels eines reversiblen oder irreversiblen Verstellmechanismus verstell- und/oder arretierbar. Reversible Verstellmechanismen sind insbesondere hinsichtlich einer späteren erforderlichen Entnahme des Implantats, beispielsweise bei Komplikationen oder Unverträglichkeiten vorteilhaft. Reversible (auch: lösbare) Verstellmechanismen können beispielsweise Stellschrauben oder dergleichen aufweisen. Irreversible Verstellmechanismen, wie etwa Rastverbindungen oder dergleichen, haben im Allgemeinen den Vorteil einer vereinfachten Herstellung. Zudem kann eine Rückbewegung der Abstützelemente zueinander insbesondere unter Axiallast in Richtung der Wirbelsäulenlängsachse effizient unterbunden werden.

Der reversible Verstellmechanismus ist in Ausgestaltung bevorzugt als Schraubgetriebe ausgeführt und weist eine in einem Gewindegang geführte Gewindespindel auf. Eine derartiges Schraubgetriebe mit Gewindespindel ist selbsthemmend ausgebildet, d. h. eine in Richtung der Hochachse bzw. der Wirbelsäulenlängsachse wirkende Axiallast erzeugt ein Schermoment, das auf die im Wesentlichen insbesondere längs der Kreisbogenkontur geführte Gewindespindel wirkt, welches die Gewindespindel blockiert und somit einer Rückbewegung der zueinander verstellten Abstützelementen entgegenwirkt. Des Weiteren erzeugt auch insbesondere die Ausrichtung der Kreisbogenkontur bezüglich der Hochachse ein auf die Gewindespindel einwirkendes Schermoment unter einer in Richtung der der Hochachse wirkenden Axiallast. Einer Rückbewegung der beiden zueinander verstellten Abstützelemente aufgrund von längs der Wirbelsäulenlängsachse wirkenden Kräften kann somit effizient entgegengewirkt werden. Zudem nimmt ein derartiges ausgeführtes Schraubgetriebe nur relativ wenig Bauraum in Anspruch, so dass ein großer Teil, beispielsweise in etwa 50% oder mehr, des Volumens des Zwischenwirbelimplantats durchlässig ausgebildet werden kann, um das bereits beschriebene Verwachsen mit Knochenmaterial zu ermöglichen. Zum Verstellen der beiden Abstützelemente zueinander wird insbesondere ein Implantationswerkzeug, welches beispielsweise ähnlich einem Schraubendreher oder Sechskantschlüssel ausgebildet ist, in das Zwischenwirbelimplantat eingeführt und die Gewindespindel in eine Drehbewegung versetzt.

In Ausgestaltungen ist der Gewindegang in einem, insbesondere ausschließlich nur in einem der beiden Abstützelemente eingebracht und weist eine Krümmung auf, die der Krümmung der Kreisbogenkontur entspricht. Auf diese Weise ist ein reversibler Verstellmechanismus angegeben, der ein kontinuierliches Verstellen der beiden Abstützelemente zueinander entlang der Kreisbogenkontur ermöglicht. Beispielsweise erstreckt sich der Gewindegang über etwa 50% der Längsausdehnung des Zwischenwirbelimplantats in Richtung der Längsachse. Der Gewindegang ist innenseitig an dem Abstützelement eingebracht und beispielsweise mit diesem einstückig ausgeführt. In anderen Ausgestaltungen wird der Gewindegang zunächst als separates Bauteil gefertigt und anschließend mit dem Abstützelement insbesondere mittels gängiger Fügetechniken verbunden.

In Ausgestaltung ist die Gewindespindel optional in einen Drehbewegung hemmenden Spindelsitz einführbar. Hierzu kann die Gewindespindel beispielsweise endseitig einen Abschnitt mit nicht rotationssymmetrischem Querschnitt aufweisen, der in einen entsprechend komplementär ausgestalteten Spindelsitz insbesondere formschlüssig einführbar ist. Beispielsweise kann die Gewindespindel mit Hilfe des bereits genannten Implantationswerkzeugs aus dem Spindelsitz herausgeschoben werden, um ein Verstellen der beiden Abstützelemente zueinander zu ermöglichen.

In Ausgestaltungen ist ein reversiblen oder irreversibler Verstellmechanismus vorgesehen, der insbesondere als Rastmechanismus ausgeführt ist. Hierzu können die beiden Abstützelemente auf einander zugewandten Seiten beispielsweise mit zueinander komplementär ausgebildeten Rastnasen, -zähnen oder dergleichen versehen sein, die eine Arretierung der beiden Abstützelemente an vorgegebenen Positionen ermöglichen.

In Ausgestaltungen des Rastmechanismus umfasst eines der Abstützelemente zumindest einen auslenkbaren Rastarm und das andere der Abstützelemente mehrere, entlang der Kreisbogenkontur zueinander beabstandete Rastnuten. Der zumindest eine auslenkbare, insbesondere federnd ausgeführte Rastarm ist dazu ausgebildet, zur Arretierung der Abstützelemente in die Rastnuten einzurasten.

In Ausgestaltungen ist ein lateraler Außenmantel des Zwischenwirbelimplantats massiv ausgebildet und weist insbesondere keine Öffnungen auf. Derartige Ausführung der erhöhten mechanischen Stabilität.

Das Zwischenwirbelimplantat besteht in bevorzugten Ausgestaltungen zumindest zum Teil, bevorzugt vollständig, aus einem Metall, einer Metalllegierung, insbesondere einer Titanlegierung, oder einem Kunststoff, insbesondere aus Polyetherketon. Besonders bevorzugt sind so genannte Grade-5 Legierungen, insbesondere Ti-6Al-4V, welches sich durch hohe Festigkeit und Beständigkeit auszeichnet. In anderen Ausgestaltungen besteht das Metall oder die Metalllegierung aus Titan, Zirkonium, oxidiertem Zirkonium, Hafnium, Platin, Rhodium, Niobium, medizinischem Edelstahl, Cobalt-Chrom-Stahl oder Tantal. Als nicht-metallische Werkstoffe, aus denen das Zwischenimplantat gefertigt sein kann, kommen des Weiteren faserverstärkte Kunststoffe, wie etwa in einer Matrix eingebettete Glas- und/oder Carbonfasern in Betracht.

Das Zwischenwirbelimplantat ist in bevorzugten Ausgestaltungen als cervicaler, thorakaler oder lumbaler Cage, insbesondere ALIF-Cage, PLIF-Cage oder TLIF-Cage, oder als künstliche Bandscheibe oder als Implantat zur Fusion von Wirbeln ausgeführt.

Das vorstehend beschriebene Zwischenwirbelimplantat kann beispielsweise unter Verwendung von herkömmlichen, insbesondere subtraktiven Herstellungsverfahren, hergestellt werden. In diesem Zusammenhang kommen insbesondere Fräsen oder andere abtragende Fertigungsmethoden, wie insbesondere das Laserschneiden und/oder der Laserablation in Betracht. Aufgrund der komplexen Geometrie des Zwischenwirbelimplantats wird dieses in bevorzugten Ausgestaltungen mittels eines generativen Herstellungsverfahrens (auch: generatives Fertigungsverfahren, additive Fertigung, 3D-Druck), insbesondere mittels selektivem Laserschmelzen, selektivem Lasersintern, Elektronenstrahlschmelzen oder Fused Filament Fabrication hergestellt. Durch die Verwendung von derartigen generativen Fertigungstechniken ist insbesondere sichergestellt, dass die Abstützelemente aus einem Stück gebildet sind. Dadurch können insbesondere mechanische Schwachstellen, die beispielsweise beim nachträglichen Fügen von mehrteiligen Bauteilen entstehen können, vermieden werden.

Für eine weitere Beschreibung der Erfindung wird auf die in den Zeichnungsfiguren gezeigten Ausführungsbeispiele verwiesen. Es zeigen in einer schematischen Darstellung:
- Fig. 1: ein mehrteiliges Zwischenwirbelimplantat gemäß einem ersten Ausführungsbeispiel in einer Explosionsdarstellung;
- Fig. 2: das in einer ersten Stellung arretierte Zwischenwirbelimplantat der Fig. 1 in einer perspektivischen Darstellung;
- Fig. 3: das in einer zweiten Stellung arretierte Zwischenwirbelimplantat der Fig. 1 in einer perspektivischen Darstellung;
- Fig. 4: das in einer dritten Stellung arretierte Zwischenwirbelimplantat der Fig. 1 in einer perspektivischen Darstellung;
- Fig. 5: das in der ersten Stellung arretierte Zwischenimplantat gemäß dem ersten Ausführungsbeispiel in einer Seitenansicht;
- Fig. 6: ein erstes Abstützelement des Zwischenimplantats gemäß dem ersten Ausführungsbeispiel in einer Seitenansicht;
- Fig. 7: eine Kontaktoberfläche des ersten Abstützelements der Fig. 6 in einer Draufsicht;
- Fig. 8: eine Innenseite des ersten Abstützelements der Fig. 6 in einer Draufsicht;
- Fig. 9: das erste Abstützelements der Fig. 6 in einer Draufsicht längs der Längsachse;
- Fig. 10: das erste Abstützelements der Fig. 6 in einer weiteren Draufsicht längs der Längsachse;
- Fig. 11: ein zweites Abstützelement des Zwischenimplantats gemäß dem ersten Ausführungsbeispiel in einer Seitenansicht;
- Fig. 12: eine Kontaktoberfläche des zweiten Abstützelements der Fig. 11 in einer Draufsicht;
- Fig. 13: eine Innenseite des zweiten Abstützelements der Fig.11 in einer Draufsicht;
- Fig. 14: das zweite Abstützelements der Fig. 11 in einer Draufsicht längs der Längsachse;
- Fig. 15: das zweite Abstützelements der Fig. 11 in einer weiteren Draufsicht längs der Längsachse;
- Fig. 16: ein Abstützelement eines zweiten Ausführungsbeispiels in einer perspektivischen Ansicht;
- Fig. 17: ein Abstützelement des zweiten Ausführungsbeispiels in einer Draufsicht;
- Fig. 18: ein Abstützelement eines dritten Ausführungsbeispiels in einer perspektivischen Ansicht;
- Fig. 19: ein Abstützelement des dritten Ausführungsbeispiels in perspektivischen Ansicht;
- Fig. 20: ein Zwischenwirbelimplantat eines vierten Ausführungsbeispiels mit einem Rastmechanismus in einer Explosionsdarstellung;
- Fig. 21: das Zwischenwirbelimplantat des vierten Ausführungsbeispiels in einer Schnittdarstellung.

Einander entsprechende Teile sind in allen Figuren mit den gleichen Bezugszeichen versehen.

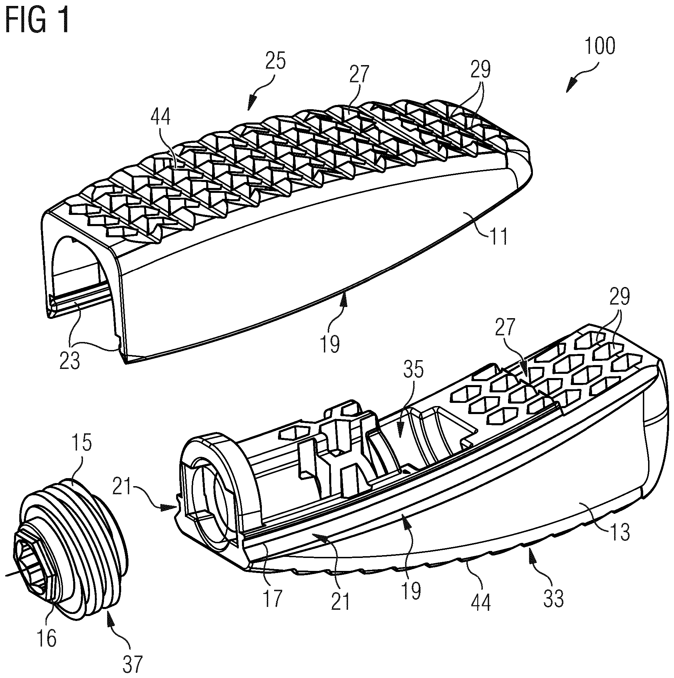

Figuren 1 bis 15 illustrieren ein lediglich exemplarisch und nicht einschränkend aufzufassendes erstes Ausführungsbeispiel des erfindungsgemäßen Zwischenwirbelimplantats 100.

Figur 1 zeigt das mehrteilige Zwischenwirbelimplantat 100 gemäß dem ersten Ausführungsbeispiel in einer Explosionsdarstellung. Das Zwischenwirbelimplantat 100 umfasst drei Bauteile: Ein erstes Abstützelement 11, ein zweites Abstützelement 13 und eine Gewindespindel 15. Diese drei Bauteile 11, 13, 15 sind im ersten Ausführungsbeispiel jeweils einstückig ausgebildet, in hierzu alternativen Ausgestaltungen können diese Bauteile 11, 13, 15 jeweils auch aus mehreren Komponenten gebildet sein, die in einem abschließenden Fertigungsschritt gefügt wurden.

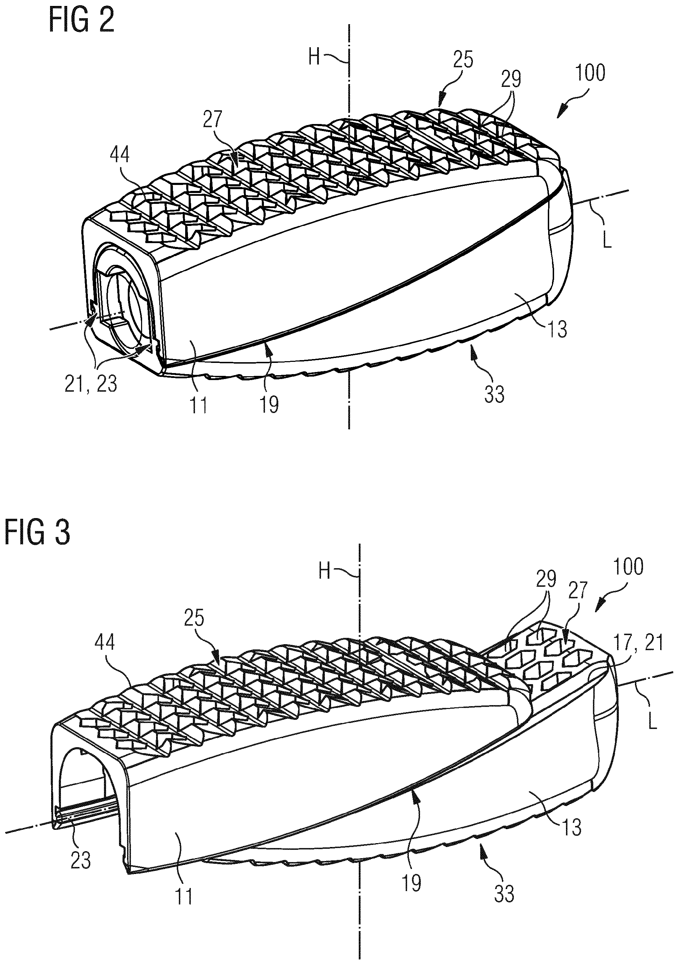

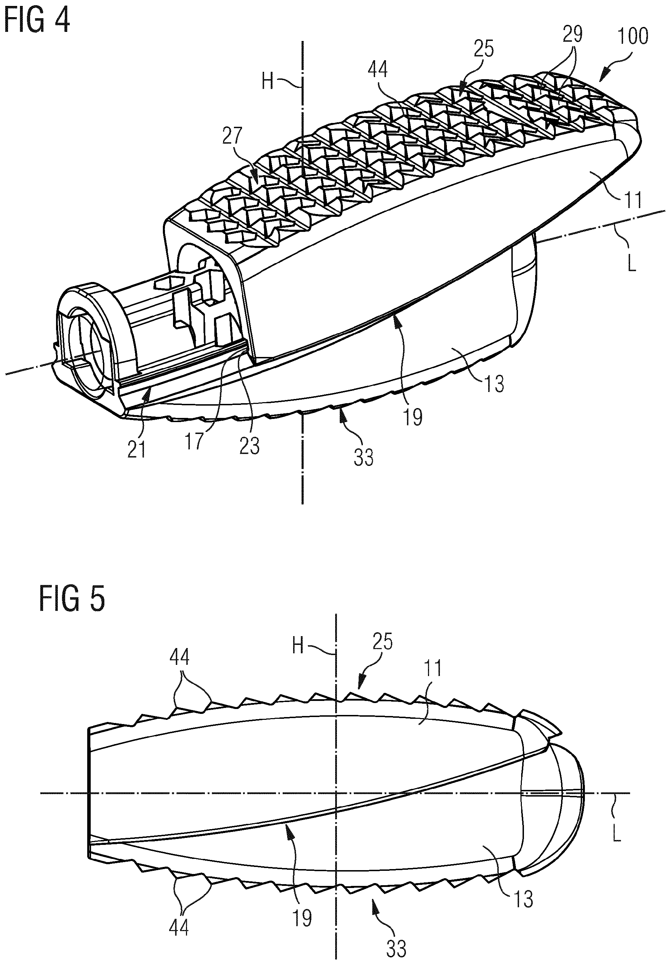

Die Abstützelemente 11, 13 bestimmen im Wesentlichen die äußere gegenständliche Gestalt des Zwischenwirbelimplantats 100, welche variierbar ist. Hierzu sind das erste und das zweite Abstützelement zueinander entlang einer Führung 17 verschiebbar gelagert. Die Führung 17 erstreckt sich längs einer Kreisbogenkontur 19 derart, dass durch Verstellen der beiden Abstützelemente 11, 13 relativ zueinander deren Ausrichtung bezüglich einer Längsachse L und einer Hochachse H, die Längsausdehnung des Zwischenwirbelimplantats 100 entlang der Längsachse L und die Höhenausdehnung in Richtung der Hochachse H verändert werden kann. Dies ist insbesondere schematisch in der perspektivischen Darstellung der Figuren 2 bis 4 illustriert. Um eine derartige Funktionalität zu ermöglichen, verläuft die Krümmung der Kreisbogenkontur 19 in der von der Hochachse H und der Längsachse L aufgespannten Ebene bzw. in einer hierzu parallel verlaufenden Ebene. Dies ist beispielsweise insbesondere aus der Seitenansicht der Figur 5 ersichtlich.

Die Führung 17 umfasst zwei Nuten 21, die sich jeweils entlang der Kreisbogenkontur 19 erstrecken und an lateral gegenüberliegenden Seiten des zweiten Abstützelements 13 eingebracht sind. Entsprechend weist das erste Abstützelement 11 Vorsprünge 23 auf, die zu den Nuten 21 komplementäre Formgebungen aufweisen und im endmontierten Zustand (vgl. insbesondere Figuren 2 bis 4) in diese eingreifen. Auf diese Weise wird die Relativbewegung der beiden Abstützelemente 11, 13 zueinander auf ein relatives Verschieben entlang der Kreisbogenkontur 19 beschränkt.

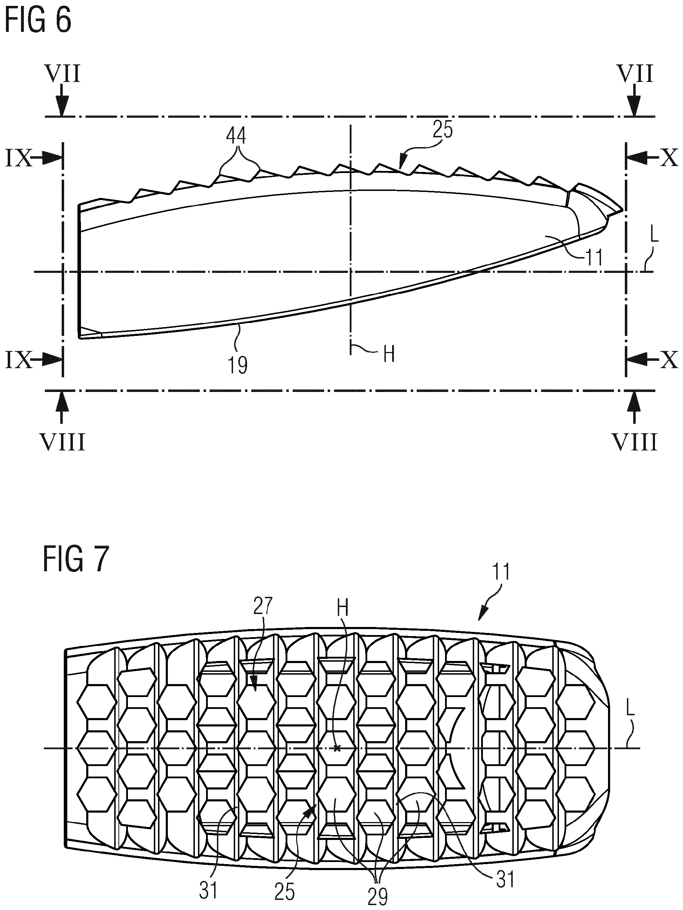

Die Gestalt des ersten Abstützelements 13 kann am besten den Figuren 6 bis 10 entnommen werden. Das erste Abstützelement 7 weist eine erste Kontaktoberfläche 25 auf, die dazu vorgesehen ist, im implantierten Zustand zumindest abschnittsweise direkt an einem Wirbelkörper anzuliegen. Wie insbesondere aus Figuren 5 oder 6 ersichtlich, weist die erste Kontaktoberfläche 25 eine gezahnte oder geriffelte Oberflächenstrukturierung mit mehreren, zueinander parallel verlaufende Rippen 44 auf. Alternativ hierzu kann die erste Kontaktoberfläche 25 auch glatt ausgeführt sein.

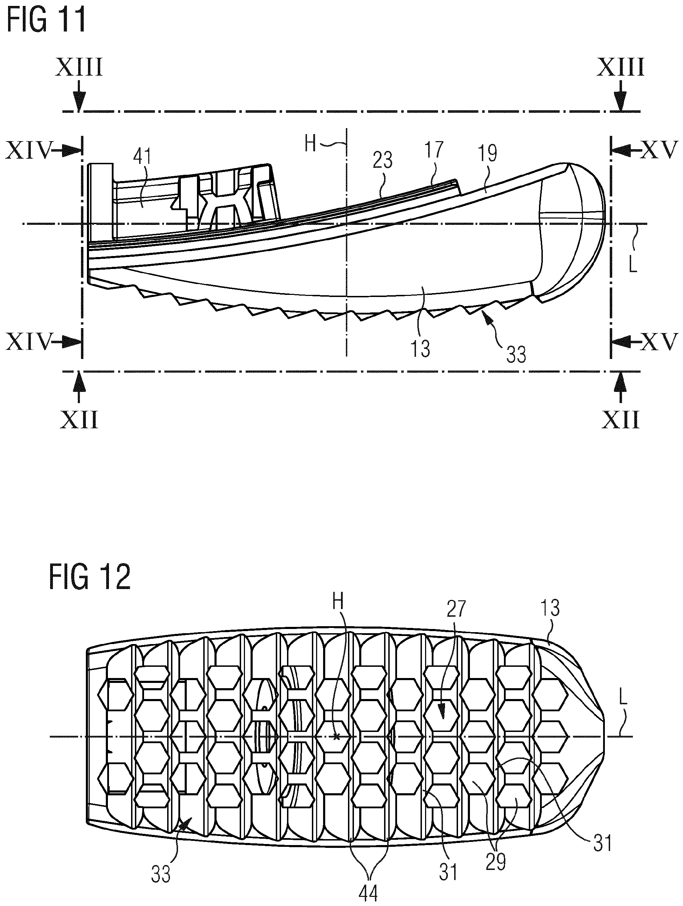

Das zweite Abstützelement 13, welches im Detail in den Figuren 11 bis 15 gezeigt ist, weist ein zweite Kontaktoberfläche 33 auf, die hinsichtlich der gegenständlichen Ausgestaltung der ersten Kontaktoberfläche 25 im Wesentlichen entspricht und ebenfalls zur Anlage an einem Wirbelkörper vorgesehen ist.

Die Kontaktoberflächen 25, 33 des ersten Ausführungsbeispiels 25 sind konvex, also jeweils nach außen gewölbt. Alternativ dazu kann die erste und/oder zweite Kontaktoberfläche 25, 33 auch plan, also eben, ausgebildet sein.

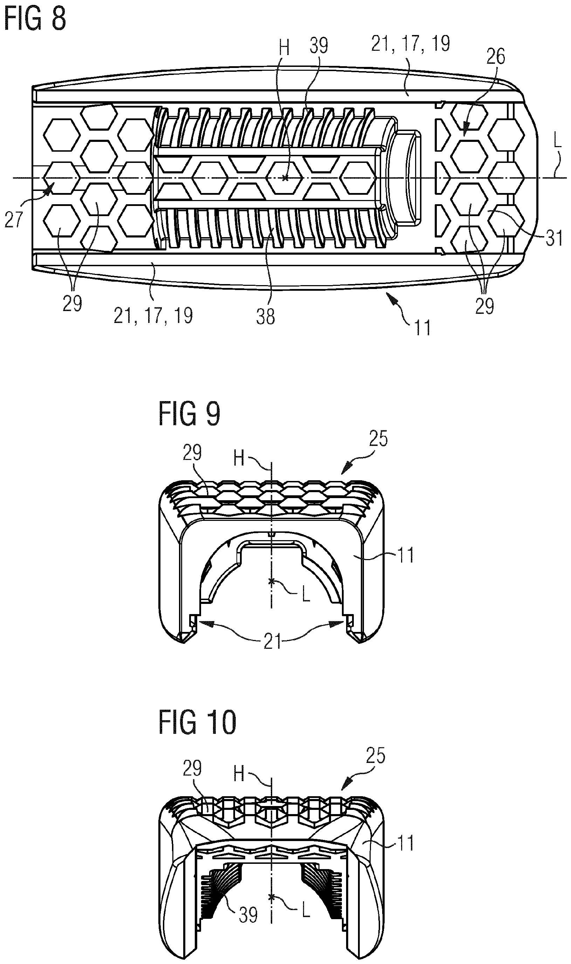

Das erste Abstützelement 11 bildet ferner einen Abschnitt einer inneren Struktur 27, die sich in Richtung der Hochachse H durch das gesamte Zwischenwirbelimplantat 100 erstreckt und von einer Vielzahl von nebeneinanderliegenden Kanälen 29 gebildet ist. Die innerhalb des ersten Abstützelemente 11 eingebrachten Abschnitte der Kanäle 29 bilden eine offene Kanalstruktur, die sowohl zur ersten Kontaktoberfläche 25 als auch zu einer ersten Innenseite 26 (vgl. insbesondere Figur 8) des ersten Abstützelements 11 hin offen sind.

Das zweite Abstützelement 13 bildet einen weiteren Abschnitt der inneren Struktur 27 und weist hierzu ebenfalls innere Kanäle 29 mit sechseckigem Querschnitt auf, die sich entlang der Hochachse H durch das zweite Abstützelement 13 erstrecken. Die Abschnitte der Kanäle 29, die sich durch das zweite Abstützelement 13 erstrecken, sind entsprechend ebenfalls zur zweiten Kontaktoberfläche 33 und zur zweiten Innenseite 34 des zweiten Abstützelements 13 hin offen.

Die von den Kanälen 29 des ersten und zweiten Abstützelements 11, 13 gebildete innere Struktur 27 bildet eine in Richtung der Hochachse H zumindest abschnittsweise durchlässige Struktur, um ein Verwachsen des Zwischenwirbelimplantats 100 mit Knochenmaterial oder das Ausfüllen mit künstlichem Knochenersatzmaterial zu begünstigen. Die innere Struktur 27 in Richtung der Hochachse H ist vorteilhafter Weise auch bei einem Verstellen der beiden Abstützelemente 11, 13 zueinander (vgl. insbesondere Figuren 2 bis 4) sicherzustellen. Kanalwände 31, welche die Kanäle 29 seitlich begrenzen, haben eine Wandstärke, die deutlich kleiner ist als der durchschnittliche Durchmesser der Kanäle 29. Dadurch wird erreicht, dass die in den ersten und zweiten Abstützelementen 11, 13 eingebrachten Kanalstrukturen für unterschiedliche Stellungen der Abstützelemente 11, 13 zueinander zumindest bereichsweise überlappen und somit fluidische Verbindungen zwischen den Kontaktoberflächen 25, 33 bereitstellen.

In dem lediglich exemplarisch dargestellten ersten Ausführungsbeispiel haben die Kanäle 29 im Wesentlichen eine hexagonale Symmetrie, entsprechend ist die innere Struktur 27 im Wesentlichen wabenförmig ausgebildet.

Die innere Struktur 27 kann in alternativen Ausgestaltungen unterschiedlich ausgeführt sein, insbesondere kann diese hinsichtlich der Geometrie und Anordnung der Kanäle 29 nahezu beliebig variieren. Vorteilhaft ist jedoch, wenn sich zumindest der überwiegende Anteil der Kanäle 29 durch das gesamte Zwischenwirbelimplantat 100 erstreckt, damit dieses möglichst vollständig mit Knochenmaterial durchwachsen oder mit Knochenersatzmaterial verfüllt werden kann.

Die offenen Kanäle 29 weisen jeweils eine Querschnittsfläche von 8.000 µm² bis 7.000.000 µm², bevorzugt eine Querschnittsfläche von 50.000 µm² bis 3.100.000 µm², insbesondere eine Querschnittsfläche von 125.000 µm² bis 570.000 µm² auf. Derartig dimensionierte Kanäle 29 begünstigen das Eindringen von Blut in die von den Kanälen 29 gebildete innere Struktur 27 in ausreichender Tiefe, um so das Verwachsen des Implantats mit den anliegenden Knochen, insbesondere Wirbelkörpern zu begünstigen.

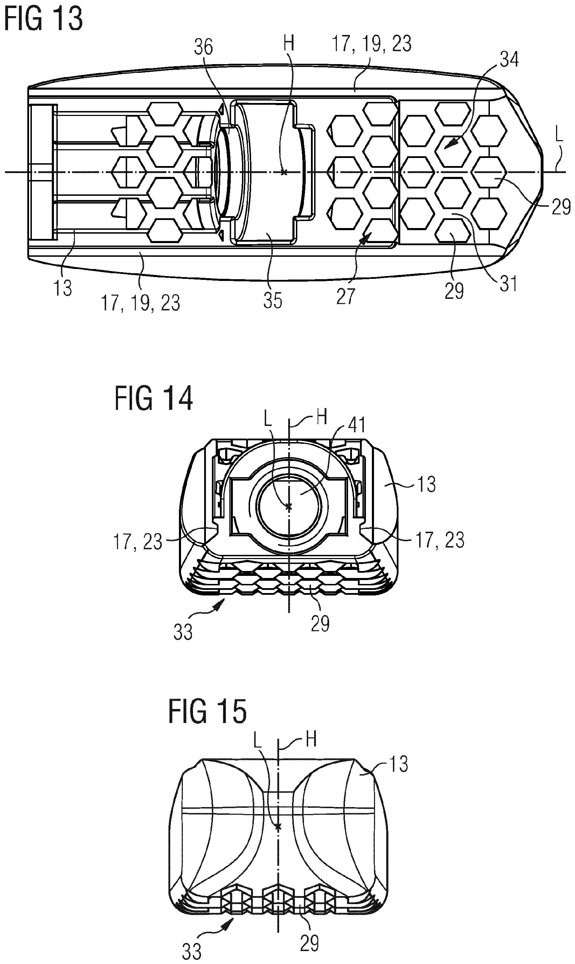

Die Gewindespindel 15 ist in eine Ausnehmung 35, welche in etwa mittig im zweiten Abstützelement 13 eingebracht ist, eingesetzt (vgl. insbesondere Figur 13). Das Außengewinde 37 der eingesetzte Gewindespindel 15 greift in einen mit einem entsprechenden Innengewinde 38 versehenen Gewindegang 39 ein, der in das erste Abstützelement 11 eingebracht ist (vgl. insbesondere Figur 8). Der Gewindegang 39 erstreckt sich in der lediglich beispielhaft dargestellten und nicht einschränkend aufzufassenden Ausführung über etwa 50% der axialen Länge des Zwischenwirbelimplantats 100.

Die eingesetzte Gewindespindel 15 kann durch ein eingesetztes Implantationswerkzeug zum Verstellen der beiden Abstützelemente 11, 13 in eine Drehbewegung versetzt werden. Das zweite Abstützelement 13 mit einer länglichen Einführöffnung 41 zum Einführen des Implantationswerkzeugs versehen, der sich im Wesentlichen axial in Richtung der Längsachse L erstreckt.

In alternativen Ausführungen ist sowohl die Ausnehmung 35 zur Aufnahme der Gewindespindel 15 als auch der Gewindegang 39 entlang der Längsachse L endseitig versetzt angeordnet.

Die Lage der Gewindespindel 15 bezüglich des zweiten Abstützelements 13 ist durch die Ausnehmung 35 -gegebenenfalls bis auf ein vorgegebenes Spiel- fixiert. Bei einer Drehung der Gewindespindel 15 läuft diese entlang des Gewindegangs 39, so dass das erste Abstützelement 11 relativ zum zweiten Abstützelement 13 entlang der Kreisbogenkontur 19 verschoben wird.

Der Gewindegang 39 ist gekrümmt ausgeführt. Insbesondere entspricht die Krümmung des Gewindegangs 39 derjenigen der Kreisbogenkontur 19. Die gekrümmte Kontur des Gewindegangs 39 verläuft somit insbesondere parallel zur Kreisbogenkontur 19.

Die Gewindespindel 15 kann endseitig eine Außenkontur 16 (vgl. insbesondere Figur 1) aufweisen, die keine kontinuierliche Drehsymmetrie hat und dazu vorgesehen ist, in einen komplementär geformten Spindelsitz 36 (vgl. insbesondere Figur 13) eingeführt zu werden, um so eine ungewollte Drehbewegung der Gewindespindel 15 zu hemmen. Der Spindelsitz 36 ist am Ende der Ausnehmung 35 und nimmt das ein Ende der Gewindespindel 15 mit der Außenkontur 16 formschlüssig auf, um insbesondere eine Rückbewegung der zueinander verstellten Abstützelemente 11, 13 unter einer in Richtung der Wirbelsäulenlängsachse wirkenden Axiallast zumindest weitgehend zu vermeiden. Die Außenkontur der Gewindespindel 15 und der Spindelsitz 35 sind zweckmäßiger Weise zueinander komplementäre und nicht rotationssymmetrisch ausgebildet. In der lediglich exemplarisch dargestellten und nicht einschränkend aufzufassenden Ausführung hat die Außenkontur 16 die Form eines Außensechskants und der Spindelsitz 36 die Form eines Innensechskants. Andere gegenständliche Ausgestaltungen sind möglich und liegen im Rahmen der Erfindung.

Die Gewindespindel 15 und der Spindelsitz sind derart ausgeführt, dass die Gewindespindel 15 aus dem Spindelsitz 36 mittels eines Implantationswerkzeugs herausgeführt bzw. herausgeschoben werden kann, damit die Gewindespindel 15 zum Verstellen der beiden Abstützelemente 11, 13 relativ zueinander gedreht werden kann.

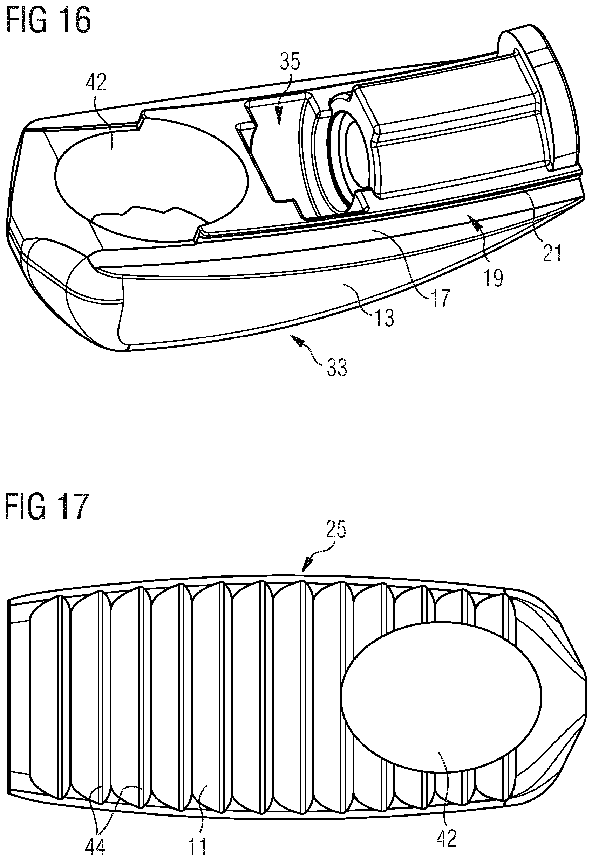

Figuren 16 und 17 zeigen eine mögliche Ausgestaltung des Zwischenwirbelimplantats 100. Figur 16 zeigt eine alternative Ausgestaltung des zweiten Abstützelements 13 in einer perspektivischen Ansicht. Figur 17 zeigt eine alternative Ausgestaltung des ersten Abstützelements 11 in einer Draufsicht. Die beiden Abstützelemente 11, 13 sind zueinander mittels eines eine Gewindespindel 15 und einen Gewindegang 39 (in Figuren 16, 17 nicht explizit dargestellt) aufweisenden Schraubgetriebes verstellbar. Die zur Aufnahme der Gewindespindel 15 vorgesehene Ausnehmung 35 ist in etwa mittig im zweiten Abstützelement 13 angeordnet.

Im Gegensatz zu dem mit Bezug auf die Figuren 1 bis 15 bereits beschriebenen ersten Ausführungsbeispiel weisen die Abstützelemente 11, 13 des in Figuren 16 und 17 dargestellten zweiten Ausführungsbeispiels keine filigrane innere Kanalstruktur auf. Das erste und das zweite Abstützelement 11, 13 weisen relative große Kavitäten 42 auf, die endseitig versetzt angeordnet sind und einen ovalen Querschnitt aufweisen und Öffnungen auf den Kontaktoberflächen 25, 33 bilden. Die von den Kavitäten 42 gebildeten Öffnungen nehmen etwas weniger als 50% der Kontaktoberfläche 25, 33 in Anspruch. Die Kontaktoberflächen 25, 33 haben eine geriffelte Oberflächenstruktur mit mehreren parallel zueinander verlaufende Rippen 44.

Die Kavitäten 42 sind im montierten Zustand des Zwischenwirbelimplantats 100 gegenüberliegende angeordnet, so dass diese zumindest bei typischen Verstellpositionen einen in Richtung der Hochachse H durchlässigen Hohlraum bilden.

Im Übrigen entspricht das zweite Ausführungsbeispiel im Wesentlichen dem bereits mit Bezug auf die Figuren 1 bis 15 beschriebenen ersten Ausführungsbeispiel, so dass auf die diesbezügliche Beschreibung verwiesen wird.

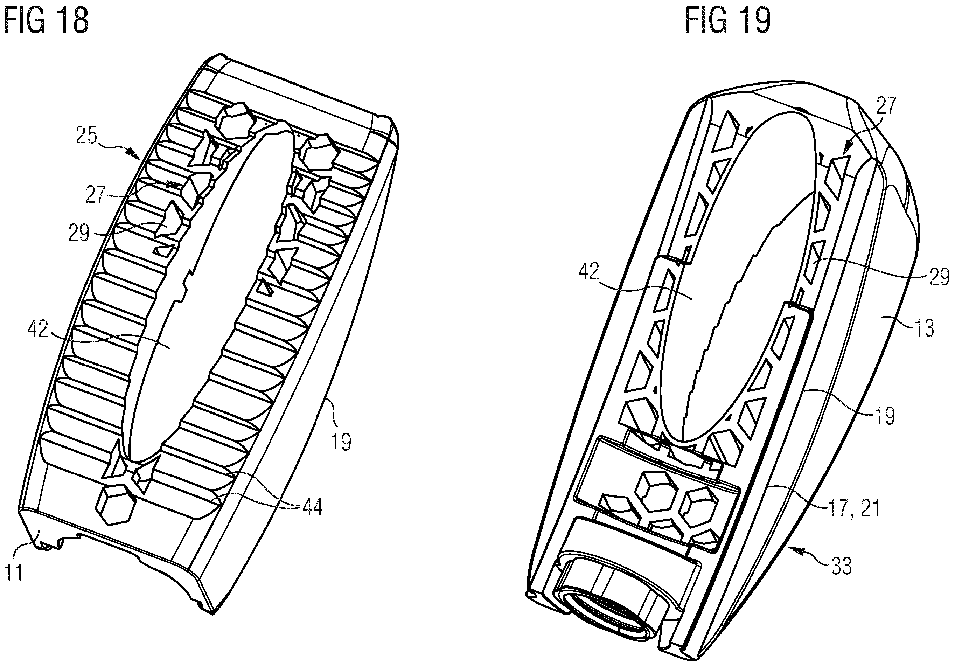

Figuren 18 und 19 zeigen ein weiteres Ausführungsbeispiel des erfindungsgemäßen Zwischenwirbelimplantats 100, dass strukturell sowohl dem ersten Ausführungsbeispiel als auch dem zweiten Ausführungsbeispiel der Figuren 17 und 16 ähnelt. Das erste und das zweite Abstützelement 11, 13 des exemplarisch dargestellten dritten Ausführungsbeispiels weisen jeweils eine längliche, im Wesentlichen mittig angeordnete Kavität 42 mit einer ovalen Gestalt auf. Die von den Kavitäten 42 gebildeten Öffnungen nehmen in etwa 30% der Kontaktoberfläche 25, 33 in Anspruch. Die beiden Kavitäten 42 des ersten und des zweiten Abstützelements 11, 13 überlappen sich bei typischen Winkelstellungen der beiden Abstützelemente 11, 13 zumindest teilweise, so dass eine entlang der Hochachse durchlässige Hohlraumstruktur gebildet ist. Zusätzlich sind kleinere innere Kanäle 29 vorgesehen, die ebenfalls ein Eindringen bzw. Einwachsen von Knochenmaterial im implantierten Zustand begünstigen.

Die Ausnehmung 35 für die (in Figuren 18 und 19 nicht explizit dargestellte) Gewindespindel 16 ist endseitig versetzt angeordnet.

Im Übrigen entspricht das dritte Ausführungsbeispiel im Wesentlichen dem bereits beschriebenen ersten Ausführungsbeispiel bzw. zweitem Ausführungsbeispiel, so dass auf die diesbezügliche Beschreibung verwiesen wird.

Die Größe der Kavitäten 42 insbesondere des zweiten und dritten Ausführungsbeispiels ist lediglich beispielhaft aufzufassen. In anderen Ausführungsbeispielen können die Kavitäten 42 größer oder kleiner ausgeführt sein und insbesondere Öffnungen auf den jeweiligen Kontaktoberflächen 25, 33 des entsprechenden Abstützelements 11, 13 bilden, die sich zumindest über 1/10 und höchstens über 3/4 der jeweiligen Kontaktoberfläche 25, 33 erstrecken.

In einem vierten Ausführungsbeispiel erfolgt das Arretieren der beiden zueinander verstellten Abstützelemente 11, 13 mittels eines irreversiblen Rastmechanismus.

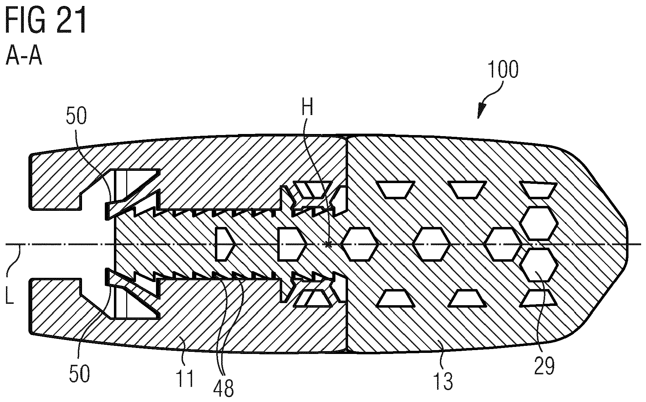

Der Rastmechanismus des vierten Ausführungsbeispiels umfasst eine Leiste 46, die die Bewegung der beiden Abstützelemente 11, 13 im Wesentlichen auf eine Translation entlang der Kreisbogenkontur 19 einschränkt und in diesem Sinne die Funktion der Führung 17 übernimmt. Die Leiste 46 erstreckt sich stegartig erhöht über einen zentralen Bereich der Innenseite des zweiten Abstützelements 13 und umfasst eine Vielzahl von lateral gegenüberliegend angeordneten Rastnuten 48, in die zur Arretierung des ersten und des zweiten Abstützelements 11, 13 an vorgegebenen Positionen entsprechend komplementär ausgebildete Rastarme 50 eingreifen können. Wie insbesondere aus der Schnittdarstellung der Fig. 21 ersichtlich, sind die Rastarme 50 innenseitig am ersten Abstützelement 11 angeordnet und in lateraler Richtung auslenkbar, so dass das erste und das zweite Abstützelement 11, 13 zueinander durch eine einfache Relativbewegung entlang der Kreisbogenkontur 19 verstellt werden können. Die Rastnuten 48 haben im Wesentlichen einen dreieckigen Querschnitt mit einer steilen und einer flachen Flanke. Die flache Flanke ist derart in Richtung der Kreisbogenkontur 17 gerichtet, dass das erste und das zweite Abstützelement 11, 13 insbesondere in Richtung zu größeren Winkeln und Abständen der Kontaktoberflächen 25, 33 zueinander verstellt werden können. Die steile Flanke der Rastnuten 48 ist entgegengesetzt orientiert, insbesondere um einer Rückbewegung der Rastarme 50 und damit der zueinander verstellten Abstützelemente 11, 13 beispielsweise unter einer in Längsrichtung der Wirbelsäule wirkenden Last entgegenzuwirken.

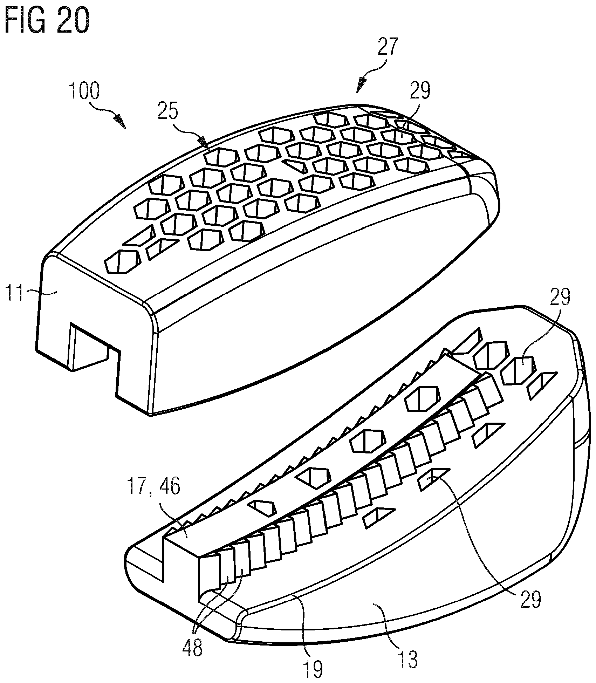

Die Kontaktoberflächen 25, 33 der Abstützelemente 11, 13 sind im Wesentlichen glatt (vgl. insbesondere Fig. 20) ausgeführt und nach außen konvex gewölbt.

Im Übrigen wird auf die bisherige Beschreibung, insbesondere mit Bezug auf die Figuren 1 bis 17, verwiesen.

Bei einem Verfahren zum Implantieren des vorstehend beschriebenen Zwischenwirbelimplantats 100 wird dieses vorzugsweise über einen dorsalen Zugang in einen Zwischenbereich zwischen zwei Wirbelkörpern der Wirbelsäule, gegebenenfalls nach Entnahme von Bandscheibenmaterial, eingesetzt. Vorteilhafter Weise wird das Zwischenwirbelimplantat 100 in einem kollabierten Zustand, wie etwa in Figur 3 schematisch illustriert, eingesetzt, damit lediglich ein minimaler Zugang erforderlich ist. Das Zwischenwirbelimplantat 100 wird in dem Zwischenbereich zwischen den beiden Wirbelkörpern derart angeordnet, dass die Hochachse H in Richtung der Wirbelsäulenlängsachse orientiert ist und die beiden Kontaktoberflächen 25, 33 jeweils in Richtung der gegenüberliegenden Wirbelkörper ausgerichtet sind. Die beiden Abstützelemente 11, 13 werden anschließend derart verstellt, dass die beiden Kontaktoberflächen 25, 33 zumindest abschnittsweise, bevorzugt über eine möglichst große Fläche an den beiden Wirbelkörpern anliegen. Vorzugsweise werden der Abstand und die Ausrichtung der beiden Kontaktoberflächen 25, 33 zueinander derart angepasst, dass diese der Ausrichtung und dem Abstand der beiden zu überbrückenden Wirbelkörper entsprechen. Hierzu wird insbesondere bei einem Zwischenwirbelimplantat 100 gemäß dem ersten Ausführungsbeispiel ein Implantationswerkzeug durch die Einführöffnung 41 in die Gewindespindel 15 einführt und anschießend gedreht.

Anschließend wird, insbesondere nach Entnahme des Implantationswerkzeugs, das Zwischenwirbelimplantat 100 optional zumindest teilweise mit künstlichem Knochenersatzmaterial verfüllt, um einem Einsinken des Zwischenwirbelimplantats 100 im natürlichen Knochenmaterial entgegenzuwirken.

Obwohl die Erfindung im Detail mit Bezug auf die bevorzugten Ausführungsbeispiele näher illustriert und beschrieben wurde, so ist die Erfindung nicht hierdurch eingeschränkt. Andere Variationen und Kombinationen können vom Fachmann hieraus abgeleitet werden, ohne vom wesentlichen Gedanken der Erfindung abzuweichen. Insbesondere sind beliebige Kombinationen von Merkmalen möglich, die mit Bezug auf verschiedene Ausführungsbeispiele und/oder Figuren beschrieben oder offenbart wurden.

Beispielsweise ist es für den Fachmann offensichtlich, den Verlauf der Kreisbogenkontur 19 hinsichtlich Krümmungsradius und/oder Neigung zu variieren, um beispielsweise die Verstellbarkeit des Zwischenwirbelimplantats 100 mit Bezug auf den Abstand und/oder die Ausrichtung der beiden Kontaktoberflächen 25, 33 zueinander im Verhältnis zur dabei einhergehenden Längenvariation anzupassen. Auch insbesondere hinsichtlich der äußeren Gestalt des Zwischenwirbelimplantats 100 sind mannigfaltige Variationen möglich, ohne vom Kerngedanken der Erfindung abzuweichen.

### Bezugszeichen liste

- 11: erstes Abstützelement
- 13: zweites Abstützelement
- 15: Gewindespindel
- 16: Außenkontur
- 17: Führung
- 19: Kreisbogenkontur
- 21: Nut
- 23: Vorsprung
- 25: Kontaktoberfläche
- 26: Innenseite
- 27: Struktur
- 29: Kanal
- 31: Kanalwand
- 33: Kontaktoberfläche
- 34: Innenseite
- 35: Ausnehmung
- 36: Spindelsitz
- 37: Außengewinde
- 38: Innengewinde
- 39: Gewindegang
- 41: Einführöffnung
- 42: Kavität
- 44: Rippen
- 46: Leiste
- 48: Rastnut
- 50: Rastarm

- 100: Zwischenwirbelimplantat

- L: Längsachse
- H: Hochachse

## Patentansprüche

1. Zwischenwirbelimplantat (100) mit zwei gegenüberliegenden, zur zumindest abschnittsweisen Anlage an Wirbelkörpern ausgebildeten Kontaktoberflächen (25, 33), die voneinander entlang einer Hochachse (H) beabstandet sind und jeweils auf relativ zueinander verstellbaren Abstützelementen (11, 13) angeordnet sind, **dadurch gekennzeichnet, dass** die Abstützelemente (11, 13) entlang einer senkrecht zur Hochachse (H) verlaufenden Längsachse (L) orientierten Kreisbogenkontur (19) mit konstantem Krümmungsradius derart zueinander verstellbar geführt sind, dass durch Verstellen der beiden Abstützelemente (11, 13) relativ zueinander entlang der Kreisbogenkontur (19) ein Abstand der beiden Kontaktoberflächen (25, 33) bezüglich der Hochachse (H) und/ oder eine Winkelstellung der beiden Kontaktoberflächen (25, 33) zueinander vorgegeben und/oder verändert werden kann.

2. Zwischenwirbelimplantat (100) nach Anspruch 1, wobei zumindest eines der Abstützelemente (11, 13) eine innere Struktur (27) mit einer Vielzahl von zur Kontaktoberfläche (25, 33) offenen Kanälen (29) umfasst, die jeweils eine Querschnittsfläche von 8.000 µm² bis 7.000.000 µm², bevorzugt eine Querschnittsfläche von 50.000 µm² bis 3.100.000 µm², besonders bevorzugt eine Querschnittsfläche von 125.000 µm² bis 570.000 µm² aufweisen.

3. Zwischenwirbelimplantat (100) nach Anspruch 2, wobei beide gegenüberliegend angeordnete Kontaktoberflächen (25, 33) mit inneren Strukturen (27) versehen sind, die jeweils eine Vielzahl von Kanälen (29) umfassen, wobei die gegenüberliegend angeordneten inneren Strukturen (27) in zumindest einem Teil des Zwischenwirbelimplantats (100) zumindest eine in Richtung der Hochachse (H) durchlässige Hohlraumstruktur bilden.

4. Zwischenwirbelimplantat (100) nach einem der Ansprüche 2 oder 3, wobei die innere Struktur wabenförmig, gitter- oder netzartig ausgebildet ist.

5. Zwischenwirbelimplantat (100) nach einem der Ansprüche 2 bis 4, wobei die Kanäle (29) einen runden oder ovalen Querschnitt oder einen Querschnitt der Form eines Polygons, insbesondere eines regelmäßigen Polygons, beispielsweise einen dreieckigen, rechteckigen, quadratischen oder hexagonalen Querschnitt aufweisen.

6. Zwischenwirbelimplantat (100) nach einem der vorherigen Ansprüche, wobei zumindest eines der Abstützelemente (11, 13) zumindest eine Kavität aufweist, die eine Öffnung auf der Kontaktoberfläche (25, 33) des zumindest einen Abstützelements (11, 13) bildet, deren Oberfläche sich zumindest über 1/10 und höchstens 3/4 der Kontaktoberfläche (25, 33) des zumindest einen Abstützelements (11, 13) erstreckt.

7. Zwischenwirbelimplantat (100) nach Anspruch 6, wobei beide gegenüberliegend angeordnete Kontaktoberflächen (25, 33) mit jeweils zumindest einer Kavität versehen sind, die derartig gegenüberliegend angeordneten sind, dass die Kavitäten in zumindest einem Teil des Zwischenwirbelimplantats (100) zumindest eine in Richtung der Hochachse (H) durchgängigen Hohlraum bilden.

8. Zwischenwirbelimplantat (100) nach einem der vorhergehenden Ansprüche, wobei zumindest eine der Kontaktoberflächen (25, 33) konvex gewölbt ist.

9. Zwischenwirbelimplantat (100) nach einem der vorhergehenden Ansprüche, wobei die beiden die Kontaktoberflächen (25, 33) aufweisenden Abstützelemente (11, 13) zueinander mittels eines reversiblen oder irreversiblen Verstellmechanismus verstell- und/oder arretierbar sind.

10. Zwischenwirbelimplantat (100) nach Anspruch 9, wobei der reversible Verstellmechanismus als Schraubgetriebe ausgeführt ist und eine in einem Gewindegang (39) geführte Gewindespindel (15) aufweist.

11. Zwischenwirbelimplantat (100) nach Anspruch 10, wobei der Gewindegang (39) in einem der Abstützelemente (11, 13) eingebracht ist und eine Krümmung aufweist, die der Krümmung der Kreisbogenkontur (19) entspricht.

12. Zwischenwirbelimplantat (100) nach Anspruch 10 oder 11, wobei die Gewindespindel (15) in einen Drehbewegung hemmenden Spindelsitz einführbar ist.

13. Zwischenwirbelimplantat (100) nach Anspruch 7, wobei der Verstellmechanismus als reversibler oder irreversibler Rastmechanismus ausgeführt ist.

14. Zwischenwirbelimplantat (100) nach Anspruch 13, wobei eines der Abstützelemente (11, 13) zumindest einen auslenkbaren Rastarm (50) und das andere der Abstützelemente (11, 13) mehrere, entlang der Kreisbogenkontur (19) zueinander beabstandete Rastnuten (48) umfasst, wobei der Rastarm (50) dazu ausgebildet ist, in die Rastnuten (48) einzurasten.

15. Verfahren zum Herstellen eines Zwischenwirbelimplantats (100) nach einem der vorhergehenden Ansprüche, wobei das Zwischenwirbelimplantat (100) mittels eines generativen Herstellungsverfahrens, insbesondere mittels selektivem Laserschmelzen, selektivem Lasersintern, Elektronenstrahlschmelzen oder Fused Filament Fabrication hergestellt wird.

## Claims

1. Intervertebral implant (100) with two opposing contact surfaces (25, 33) provided for at least partial contact with vertebral bodies, which are spaced apart from one another along a vertical axis (H) and are each arranged on support elements (11, 13) adjustable relative to one another, **characterised in that** the support elements (11, 13) are oriented perpendicularly to the vertical axis (H) along a circular arc contour (19) having a constant radius of curvature and are adjustably guided relative to one another in such a way that by adjusting the two supporting elements (11, 13) relative to one another along the circular arc contour (19), a distance between the two contact surfaces (25, 33) relative to the vertical axis (H) and/or an angular position of the two contact surfaces (25, 33) relative to one another can be specified and/or changed.

2. Intervertebral implant (100) according to claim 1, wherein at least one of the supporting elements (11, 13) comprises an inner structure (27) with a plurality of channels (29) open towards the contact surface (25, 33), each of which has a cross-sectional area of 8,000 µm² to 7,000,000 µm², preferably a cross-sectional area of 50,000 µm² to 3,100,000 µm², particularly preferably a cross-sectional area of 125,000 µm² to 570,000 µm².

3. Intervertebral implant (100) according to claim 2, wherein both oppositely arranged contact surfaces (25, 33) are provided with internal structures (27), each comprising a plurality of channels (29), wherein the oppositely arranged internal structures (27) in at least a part of the intervertebral implant (100) form at least one cavity structure permeable in the direction of the vertical axis (H).

4. Intervertebral implant (100) according to one of claims 2 or 3, wherein the inner structure is honeycombed, grid-like or net-like.

5. Intervertebral implant (100) according to one of claims 2 to 4, wherein the channels (29) have a round or oval cross-section or a cross-section of the shape of a polygon, in particular a regular polygon, for example a triangular, rectangular, square or hexagonal cross-section.

6. Intervertebral implant (100) according to one of the previous claims, wherein at least one of the support elements (11, 13) has at least one cavity forming an opening on the contact surface (25, 33) of the at least one support element (11, 13), the surface of which extends over at least 1/10 and at most 3/4 of the contact surface (25, 33) of the at least one support element (11, 13).

7. Intervertebral implant (100) according to claim 6, wherein both oppositely arranged contact surfaces (25, 33) are each provided with at least one cavity, which are arranged opposite each other in such a way that the cavities in at least a part of the intervertebral implant (100) form at least one continuous cavity in the direction of the vertical axis (H).

8. Intervertebral implant (100) according to one of the preceding claims, wherein at least one of the contact surfaces (25, 33) is convexly curved.

9. Intervertebral implant (100) according to one of the preceding claims, wherein the two support elements (11, 13) having the contact surfaces (25, 33) can be adjusted and/or locked relative to one another by means of a reversible or irreversible adjustment mechanism.

10. Intervertebral implant (100) according to claim 9, wherein the reversible adjustment mechanism is designed as a screw mechanism and has a threaded spindle (15) guided in a thread (39).

11. Intervertebral implant (100) according to claim 10, wherein the thread (39) is inserted in one of the support elements (11, 13) and has a curvature which corresponds to the curvature of the circular arc contour (19).

12. Intervertebral implant (100) according to claim 10 or 11, wherein the threaded spindle (15) can be inserted into a spindle seat inhibiting rotational movement.

13. Intervertebral implant (100) according to claim 7, wherein the adjustment mechanism is designed as a reversible or irreversible locking mechanism.

14. Intervertebral implant (100) according to claim 13, wherein one of the supporting elements (11, 13) comprises at least one deflectable detent arm (50) and the other of the supporting elements (11, 13) comprises several detent grooves (48) spaced apart from one another along the circular arc contour (19), wherein the detent arm (50) is designed to engage in the detent grooves (48).

15. Method for manufacturing an intervertebral implant (100) according to one of the preceding claims, wherein the intervertebral implant (100) is manufactured by means of a generative manufacturing process, in particular by means of selective laser melting, selective laser sintering, electron beam melting or fused filament fabrication.

## Revendications

1. Implant (100) intervertébral ayant deux surfaces (25, 33) de contact opposées qui sont constituées pour se mettre, au moins par endroit, sur des corps vertébraux, qui sont à distance l'une de l'autre suivant un axe (H) vertical et qui sont disposées chacune sur des éléments (11, 13) d'appui déplaçable relativement l'un par rapport à l'autre, **caractérisé en ce que** les éléments (11, 13) d'appui sont guidés, en pouvant se déplacer l'un par rapport à l'autre, suivant un contour (19) en arc de cercle, de rayon de courbure constant, orienté suivant un axe (L) longitudinal s'étendant perpendiculairement à l'axe (H) horizontal de manière à ce que, par le déplacement des deux éléments (11, 13) d'appui l'un par rapport à l'autre suivant le contour (19) en arc de cercle, une distance des deux surfaces (25, 33) de contact relative à l'axe (H) vertical et/ou une position angulaire des deux surfaces (25, 33) de contact l'une par rapport à l'autre puisse être prescrite et/ou modifiée.

2. Implant (100) intervertébral suivant la revendication 1, dans lequel au moins l'un des éléments (11, 13) d'appui comprend une structure (27) intérieure ayant une pluralité de canaux (29) ouverts vers la surface (25, 33) de contact, qui ont chacun une surface de section transversale de 8.000 µm² à 7.000.000 µm², en ayant de préférence, une surface de section transversale de 50.000 µm² à 3.100.000 µm², d'une manière particulièrement préférée, une surface de section transversale de 125.000 µm² à 570.000 µm².

3. Implant (100) intervertébral suivant la revendication 2, dans lequel les deux surfaces (25, 33) de contact opposées sont pourvues de structures (27) intérieures, qui comprennent chacune une pluralité de canaux (29), les structures (27) intérieures opposées formant dans au moins une partie de l'implant (100) intervertébral au moins une structure d'espace vide perméable dans la direction de l'axe (H) vertical.

4. Implant (100) intervertébral suivant l'une des revendications 2 ou 3, dans lequel la structure intérieure est constituée en forme de nid d'abeille, à la manière d'une grille ou d'un réseau.

5. Implant (100) intervertébral suivant l'une des revendications 2 à 4, dans lequel les canaux (29) ont une section transversale circulaire ou ovale ou une section transversale de la forme d'un polygone, notamment d'un polygone régulier, par exemple une section transversale triangulaire, rectangulaire, carrée ou hexagonale.

6. Implant (100) intervertébral suivant l'une des revendications précédentes, dans lequel au moins l'un des éléments (11, 13) d'appui a au moins une cavité, qui forme une ouverture sur la surface (25, 33) de contact du au moins un élément (11, 13) d'appui, dont la surface s'étend sur au moins le 1/10ème et au plus les 3/4 de la surface (25, 33) de contact du au moins un élément (11, 13) d'appui.

7. Implant (100) intervertébral suivant la revendication 6, dans lequel les deux surfaces (25, 33) de contact opposées sont pourvues chacun d'au moins une cavité, qui sont disposées en étant opposées de façon à ce que les cavités dans au moins une partie de l'implant (100) intervertébral forment au moins un espace vide perméable dans la direction de l'axe (H) vertical.

8. Implant (100) intervertébral suivant l'une des revendications précédentes, dans lequel au moins l'une des surfaces (25, 33) de contact est incurvée de manière convexe.

9. Implant (100) intervertébral suivant l'une de revendications précédentes, dans lequel les deux éléments (11, 13) d'appui ayant les surfaces (25, 33) de contact peuvent être déplacés et/ou bloqués, l'un par rapport à l'autre au moyen d'un mécanisme de réglage réversible ou irréversible.

10. Implant (100) intervertébral suivant la revendication 9, dans lequel le mécanisme de réglage réversible est réalisé sous la forme d'un engrenage hélicoïdal et a une broche (15) filetée guidée dans un pas de vis (39).

11. Implant (100) intervertébral suivant la revendication 10, dans lequel le pas de vis (39) est mis dans l'un des éléments (11, 13) d'appui et a une courbure qui correspond à la courbure du contour (19) en arc de cercle.

12. Implant (100) intervertébral suivant la revendication 10 ou 11, dans lequel la broche (15) filetée peut être insérée dans un siège de broche empêchant un mouvement de rotation.

13. Implant (100) intervertébral suivant la revendication 7, dans lequel le mécanisme de réglage est réalisé sous la forme d'un mécanisme d'encliquetage réversible ou irréversible.

14. Implant (100) intervertébral suivant la revendication 13, dans lequel l'un des éléments (11, 13) d'appui comprend au moins un bras (50) d'encliquetage pouvant être sorti et l'autre des éléments (11, 13) d'appui plusieurs rainures (48) d'encliquetage à distance les unes des autres suivant le contour en arc de cercle, le bras (50) d'encliquetage étant constitué pour s'encliqueter dans les rainures (48) d'encliquetage.

15. Procédé de fabrication d'un implant (100) intervertébral suivant l'une des revendications précédentes, dans lequel on fabrique l'implant (100) intervertébral au moyen d'un procédé de fabrication génératif, notamment au moyen d'une fusion laser sélective, d'un frittage laser sélectif, d'une fusion par faisceau d'électrons ou d'une fabrication par filament fondu.
